# EUROPÄISCHE PATENTSCHRIFT

(11) **EP 0 403 946 B1**
(45) Veröffentlichungstag und Bekanntmachung des Hinweises auf die Patenterteilung: **30.03.1994**
(21) Anmeldenummer: 90111173.2
(22) Anmeldetag: 13.06.1990
(51) Int. Cl.: G01N 33/34

(54) **Verfahren zur Vorbereitung von Papiersorten für eine Bestimmung der Spannungs-Dehnungs-Charakteristik**
Method for preparing different types of paper to determine their stress-strain characteristic
Méthode de préparation de différents types de papier pour la détermination de leur caractéristique effort-contrainte

(30) Priorität: 20.06.1989 DE 3920101
(43) Veröffentlichungstag der Anmeldung: 27.12.1990
(73) Patentinhaber: Koenig & Bauer Aktiengesellschaft, 97080 Würzburg (DE)
(72) Erfinder: Glöckner, Erhard Herbert, D-8701 Eibelstadt (DE); Pfister, Hans Bernhard, D-8700 Würzburg (DE)

(56) Entgegenhaltungen:
- EP-A- 0 344 554
- DE-C- 400 983
- US-A- 4 055 071
- ZEITSCHRIFT FÜR INSTRUMENTENKUNDE, Band 74, Heft 11, Nov. 1966; D. OCKERT, Seiten 354-358#

## Beschreibung

Die Erfindung betrifft ein Verfahren nach dem Oberbegriff des Anspruchs 1.

Für den Papierlauf in Rollenrotationsdruckmaschinen ist die Kenntnis der Spannungs-Dehnungs-Charakteristik des Papieres von großer Wichtigkeit. Im Flexo- und vorallem im Offsetdruck wird z.B. bis zu acht Mal Wasser beim Durchlauf der Papierbahn durch die Druckwerke auf die Papierbahn aufgebracht. Dadurch ändern sich die mechanischen Eigenschaften des Papiers, d. h. die Spannungs-Dehnungs-Charakteristik wird sich, je nach der verwendeten Papiersorte, mehr oder weniger stark ändern. Mit der Veränderung der Spannungs-Dehnungs-Charakteristik des Papieres wird naturgemäß auch der Papierlauf der Papierbahn durch die Druckmaschine beeinflußt.

Reproduzierbare Aussagen über den Wasserfeuchteeinfluß auf die mechanischen Eigenschaften des Papiers waren bis jetzt nur unter großem Aufwand ausschließlich in einer Klimakammer möglich. Zur Messung wurden in Länge und Breite gleiche Papierstreifen (= Meßstreifen) in eine Zugfestigkeitsprüfmaschine eingespannt und das Zerreißschaubild (Spannungs-Dehnungs-Diagramm) aufgezeichnet. Schnelle, reproduzierbare Messungen am nicht in der Klimakammer gelagerten Papier waren praktisch unmöglich. Ursache dieser Nichtreproduzierbarheit sind die unterschiedlichen Feuchtigkeitsgehalte der zu messenden Papierstreifen.

Der Erfindung liegt die Aufgabe zugrunde, ein Verfahren zu schaffen, das reproduzierbare Spannungs-Dehnungs-Messungen an Papier durchzuführen ermöglicht.

Diese Aufgabe wird erfindungsgemäß durch den kennzeichnenden Teil des Patentanspruches 1 gelöst.

Die mit der Erfindung erzielbaren Vorteile bestehen insbesondere darin, daß die zu messenden Papierstreifen einfach und schnell auf einen vorwählbaren Feuchtigkeitsgehalt gebracht werden können. Dadurch ist es möglich, das Spannungs-Dehnungsverhältnis der Papierstreifen unter nahezu gleichen Bedingungen zu messen. Die Messungen müssen nicht mehr in aufwendigen Klimakammern durchgeführt werden. Außerdem kann die Messung ohne lange Vorbereitungszeit der Messung durchgeführt werden.

Es können nicht nur niedrige wasserfeuchtigkeitsgehalte der Papiermeßstreifen auf einen höheren Wasserfeuchtigkeitsgehalt gebracht werden, d.h. auf bzw. annähernd auf den höheren prozentualen Wasseranteil des Lösungsmittel-Wasser-Gemisches, sondern auch höhere Wasserfeuchtigkeitsgehalte im Papiermeßstreifen auf einen niedrigeren Wasserfeuchtigkeitsgehalt, d.h. auf den niedrigeren prozentualen Wasseranteil des Lösungsmittel-Wasser-Gemisches gebracht werden.

Es werden weder die Zellulosestränge gespalten noch nennenswert anorganische Salze aus dem Papier gelöst.

Das erfindungsgemäße Verfahren wird nachfolgend beschrieben.

Aus einem Papierbahn- oder Bogenstück werden Papiermeßstreifen von bestimmter, jeweils konstanter Länge und Breite ausgeschnitten. Diese Papiermeßstreifen (= Papierprobe) werden mit einem homogenen, nichtemulgierten Lösungsmittel-Wasser-Gemisch von genau eingestellter Konzentration getränkt, vorzugsweise bis zur Sättigung der Flüssigkeitsaufnahme. Es eignen sich z.B. folgende Lösungsmittel (= Verfahrenslösungsmittel), vorzugsweise Azeton, aber auch: Propargylaldehyd, Formaldehyddimethylacetal, Essigsäuremethylester, Trimethylenoxid, Ethylpropylether, Zweimethylpropanal, Glyoxal, Methylisonitril.

In der folgenden Beschreibung wird stellvertretend für alle oben aufgeführten Lösungsmittel Azeton genannt.

Es wird ein Azeton/Wasser-Gemisch im Verhältnis bis zu einem Wasseranteil von 15 % (Massenprozent) eingestellt. Das Azeton-/Wasser-Gemisch ist leicht mittels Pipette, die z.B. auf 0,01 ml genau ist, einzustellen. Das Azeton-/Wasser-Gemisch wird in ein Behältnis in ausreichender Höhe eingefüllt. Anschließend werden die Papiermeßstreifen - die z.B. eine Länge von 18 cm und eine Breite von 1 cm haben - in das Flüssigkeitsgemisch so hineingelegt, daß der Papiermeßstreifen vollständig in das Feuchtigkeitsgemisch eintaucht. Das Behältnis wird unverzüglich nach Einlegen der Papiermeßstreifen oben mit einem abdichtenden Deckel verschlossen. Nach einer genau vorherbestimmten Verweilzeit tu (z.B. 20 Sekunden) im Flüssigkeitsgemisch-Bad wird der Papierstreifen aus dem Bad genommen und die überflüssige Flüssigkeit vom Streifen abgestreift. Danach läßt man den getränkten Papierstreifen eine genau definierte Zeitspanne ts (z.B. 25 Sekunden) an der Umgebungsluft, bis das in den Papiermeßstreifen eingedrungene Lösungsmittel ganz oder weitestgehend verdunstet ist.

Unmittelbar darauf wird der Papiermeßstreifen in eine Zugfestigkeits-Prüfmaschine eingespannt und die Zugfestigkeit-Dehnungs-Messung begonnen, wobei das Spannungs-Dehnungs-Diagramm aufgezeichnet und anschließend ausgewertet wird.

Während der Zeit des Verweilens des Papiermeßstreifens im Meßraum, z.B. bei Raumtemperatur, verdunstet das Lösungsmittel, z.B. Azeton, nahezu völlig aus dem Papierstreifen. Es bleibt im Papier eine gleichmäßige Feuchte zurück, die einem gewünschten Wert entspricht und für jeden Meßstreifen einer Papiersorte annähernd gleich ist. Je nach gewünschtem Wasserfeuchtegehalt im Papiermeßstreifen wird der Anteil des Wassers im Lösungsmittel festgelegt. Ein besonders günstiger Bereich ist ein Wasseranteil in dem Gemisch zwischen 4 und 15 % (Masseprozente), wobei die verschiedenen Lösungsmittel mit unterschiedlichen Mengen von Wasser mischbar sind, ohne eine Emulsion mit diesem zu bilden.

Das erfindungsgemäße Verfahren wurde stellvertretend für weitere, geeignete, flüssige Lösungsmittel unter Verwendung von Azeton beschrieben. Prinzipiell sind alle Lösungsmittel geeignet, deren Siedepunkt mindestens 30°Celsius niedriger ist als der des Wassers, in denen wenigstens 4 % (Masseprozente) Wasser gelöst werden können, die mit dem Lösungsmittel (Masseanteil) bei dem gewünschten Wassergehalt keine Emulsion bilden, die Zellulosestränge des Papiers nicht angreifen und die anorganischen Bestandteile des Papiers nicht nennenswert lösen.

## Patentansprüche

1. Verfahren zur Vorbereitung von Papiersorten für eine anschließende Messung, z.B. einer Spannungs-/Dehnungs-Kennlinie einer Papiersorte, dadurch gekennzeichnet, daß die zu messende Papierprobe mit einem nichtemulgierten Lösungsmittel-Wasser-Gemisch bis vorzugsweise zur Sättigung der Gemischaufnahme in der Papierprobe getränkt wird, daß nach einer festgelegten Zeitspanne (tv) des Verdunstens des Lösungsmittels eine Bestimmung der Spannungs-Dehnungs-Kennlinie durchgeführt wird, wobei das Lösungsmittel einen Siedepunkt aufweist, der mindestens 30° Celsius niedriger als der des Wassers ist, und das Lösungsmittel-Wasser-Gemisch während des Tränkens der Papierprobe sich im nichtemulgierten Zustand befindet.

2. Verfahren nach Anspruch 1, dadurch gekennzeichnet, daß das Lösungsmittel wahlweise Azeton, Propargylaldehyd, Formaldehyddimethylacetal, Essigsäuremethylester, Trimethylenoxid, Ethylpropylether, Zweimethylpropanal. Glyoxal, Methylisonitril, ist.

## Claims

1. Method of preparing paper types for subsequent measurement, e.g. of a tension/expansion characteristic of a paper type, characterized in that the paper sample to be measured is impregnated with a non-emulsified solvent/water mixture until preferably the mixture is absorbed to saturation point in the paper sample, in that after a fixed time span (tv) of evaporation of the solvent a determination of the tension/expansion characteristic is carried out, the solvent having a boiling point which is at least 30° Celsius lower than that of water, and the solvent/water mixture being in the non-emulsified state during the impregnation of the paper sample.

2. Method according to Claim 1, characterized in that the solvent is optionally acetone, propargylaldehyde, formaldehyde dimethylacetal, methyl acetate, trimethylene oxide, ethylpropyl ether, 2-methyl propanal, glyoxal, methyl isonitrile.

## Revendications

1. Méthode de préparation de différentes sortes de papier en vue d'effectuer ensuite une mesure, par exemple d'une caractéristique contrainte/extension d'une sorte de papier, caractérisée en ce que l'échantillon de papier à mesurer est imbibé avec un mélange solvant-eau non émulsionné, jusqu'à de préférence obtenir la saturation de la captation du mélange dans l'échantillon de papier, en ce qu'au bout d'un intervalle de temps (tv) prédéterminé, au cours duquel a lieu l'évaporation du solvant, on effectue une détermination de la caractéristique contrainte/extension, le solvant présentant un point d'ébullition inférieur d'au moins 30° Celsius à celui de l'eau, et le mélange solvant-eau se trouvant à un état non émulsionné pendant l'imprégnation de l'échantillon de papier.

2. Méthode selon la revendication 1, caractérisée en ce que le solvant est, au choix, l'acétone, l'aldéhyde de propargyle, le diméthylacétal de formaldéhyde, le méthylester d'acide acétique, le trioxyde de méthylène, l'éthylproyléther, le bi-propanal de méthyle, le glyoxal, l'isonitrile de méthyle.
